Europäisches Patentamt

(19) European Patent Office

Office européen des brevets

(11) Publication number: **0 089 948**
**B1**

(12) **EUROPEAN PATENT SPECIFICATION**

(45) Date of publication of patent specification: **14.08.85**

(21) Application number: **81902743.4**

(22) Date of filing: **05.10.81**

(86) International application number:
**PCT/GB81/00218**

(87) International publication number:
**WO 83/01199 14.04.83 Gazette 83/09**

(51) Int. Cl.⁴: **A 61 K 45/06,** A 61 K 31/54,
A 61 K 31/55, A 61 K 31/505,
A 61 K 33/00 // (A61K31/55,
31:54, 31:505, 31:405, 33:00)

(54) **PHARMACEUTICAL COMPOSITIONS.**

(43) Date of publication of application:
**05.10.83 Bulletin 83/40**

(45) Publication of the grant of the patent:
**14.08.85 Bulletin 85/33**

(84) Designated Contracting States:
**AT CH DE FR LI LU NL SE**

(56) References cited:
**DE-A-1 949 774**
**GB-A-2 072 504**

**Remington's Pharmaceutical Science, 1975, pp.
949-950**

(73) Proprietor: **COPPEN, Alec James**
**5 Walnut Close Epsom**
**Surrey KT18 5JL (GB)**

(72) Inventor: **COPPEN, Alec James**
**5 Walnut Close Epsom**
**Surrey KT18 5JL (GB)**

(74) Representative: **Marshall, Monica Anne et al**
**GALLAFENT & CO. 8 Staple Inn**
**London WC1V 7QH (GB)**

Courier Press, Leamington Spa, England.

# Description

This invention relates to anti-depressant or antimanic pharmaceutical compositions containing folic acid.

Folic acid is a vitamin widely distributed in, especially, green vegetables, yeast and liver. An ordinary Western diet for normal people generally supplies just enough folic acid to meet daily requirements. Folic acid deficiency has been observed to result in anaemia and the acid has been used for some time as a hematopoietic vitamin. In this connection it is known to administer synthetic folic acid (5 mg) orally; 10 to 30 mg daily are usually given. In addition, to prevent deficiency occurring during pregnancy when there is an increased folic acid requirement, folic acid is often administered during pregnancy.

More recently it has been observed that a substantial proportion of patients suffering from neuropsychiatric disorders have low serum folate concentrations. Treatment of the disorders by use of conventional doses of folic acid have been suggested (see "Dementia and Folate Deficiency", Strachen and Henderson, Quarterly Journal of Medicine, New Series XXXVI, No. 142, April 1967, page 189; "Effects of Folic Acid on the Mental State and Fit-Frequency of Drug-Treated Epileptic Patients", Reynolds, The Lancet, May 1967, page 106; "Folate-Responsive Neuropathy: Report of 10 Cases", Manzoor and Runcie, British Medical Journal, 15th May 1976, page 1176; and "Neurological Disorders Responsive to Folic Acid Therapy", Botez et al, CMA Journal August 7 1976, Volume 115, page 217). In all cases patients were treated with folic acid supplements at the conventional rate used in the field of haematology. In addition Reynolds et al ("Folate Deficiency in Depressive Illness", Brit. J. Psychiatry 1970, 117, 287) studied the serum folate level in patients with depressive illness and while a relationship between folic acid deficiency and depression was reported the writers were unable to elucidate it.

It is generally agreed that there is a wealth of evidence for the neurotransmitter 5-hydroxytryptamine (5-HT) being essential for the normal maintenance of mood. It is recognised that commonly used antidepressants, whose mode of action is understood, generally act by manipulating brain amines including in most cases 5-HT.

Botez et al ("Folate Deficiency and Decreased Brain 5-Hydroxytryptamine Synthesis in Man and Rat", Nature, Volume 278, 8th March 1979 182), found that brain 5-HT synthesis is reduced in both man and animals which are folate deficient. However administration of folic acid to provide a folate excess, as in conventional therapy, was ineffective in restoring brain 5-HT to normal. Only replacement of folate by folic acid in normal dietary quantities was found to restore 5-HT synthesis to normal.

Compositions comprising the anti-depressant l-tryptophan and, among other ingredients, folic acid have been disclosed in DE—A—1949774.

According to the present invention there is provided a pharmaceutical composition comprising folic acid and an anti-depressant or anti-manic drug other than tryptophan, the folic acid and the drug being present at a ratio equal to 100 to 300 μg folic acid to the daily dose of the drug.

The normal daily intake of folic acid, as recommended by DHSS Report on Health and Social Subjects No. 15, is generally 200 μg per day. Thus the composition according to the present invention contains folic acid in the ratio of approximately the normal daily dietary requirement and preferably 200 μg, of folic acid per daily dose of the drug.

The daily dose of the drug is of course that of accepted medical practice and will vary from drug to drug. If for example the composition is made up in the form of a tablet or capsule containing a proportion of the daily dose of drug, a number of the tablets or capsules to be taken daily, then, in accordance with the present invention, the composition should contain the same proportion of the normal daily dietary requirement of folic acid. Thus if two tablets are to be taken daily, the tablets will each contain one half of the normal daily dietary requirement of folic acid.

While it is not wished to be bound by the mode of action of the present composition, it is believed that the provision of folic acid enables the synthesis of 5-HT to be returned to normal and thus increasing the effectiveness of the anti-depressant or anti-manic drug.

The drug may be a tricyclic anti-depressant or other anti-depressant such as imipramine, chlominipramine, zimelidine, norzimelidine, nortriptyline, protriptyline, amitriptyline, fluxpenthixol, butriptyline, maprotiline, nomifensine, prothiaden, desimipramine, iprindole, viloxazine, dothiepin, doxepin, trimipramine, mianserin, or a monoamineoxidase inhibitor such as phenelzine, isocarboxizid, iproniazid, tranylcypromine. The folic acid can also be used with lithium carbonate (given usually to treat mania and to prevent recurrences of depression and mania), in normal or slow or sustained release form.

Anti-manic drugs which can be used in the composition of the present invention include phenothiazines and other anti-manic drugs such as chlorpromazine, flupenthixol, haloperidol, thioridazine, fluphenazine, thiothixene, pimozide, promazine and trifluoperazine.

# Claims

1. A pharmaceutical composition comprising folic acid and an anti-depressant or anti-manic drug other than tryptophan, the folic acid and the drug being present at a ratio equal to 100 to 300 μg folic acid to the daily dose of the drug.

2. A composition according to claim 1 which contains the folic acid and drug at a ratio equal to about 200 µg of folic acid to the daily dose of the drug.

3. A composition according to claim 1 or 2 wherein the drug is imipramine, chlomimipramine, zimelidine, nortriptyline, protriptyline, amitriptyline, fluxpenthixol, butriptyline, maprotiline, nomifensine, prothiaden, fluphenazine, desimipramine, iprindole, viloxazine, dothiepin, doxepin, trimipramine, mianserin, norzimelidine, phenelzine, isocarboxizid, iproniazid, tranylcypromine, lithium carbonate, chlorpromazine, flupenthixol, haloperidol, thioridazine, thiothixene, pimozide, promazine or trifluoperazine.

4. A composition according to any one of claims 1 to 3 which is in the form of a tablet or capsule.

## Patentansprüche

1. Pharmazeutische Zusammensetzung, dadurch gekennzeichnet, daß sie Folsäure und ein Antidepressivum oder ein antimanisches Medikament verschieden von Tryptophan umfaßt, wobei die Folsäure und das Medikament in einem Verhältnis gleich 100 bis 300 µg Folsäure zur täglichen Dosierung des Medikaments vorhanden sind.

2. Zusammensetzung nach Anspruch 1, dadurch gekennzeichnet, daß sie Folsäure und das Medikament in einem Verhältnis gleich etwa 200 µg der Folsäure zur täglichen Dosierung des Medikaments enthält.

3. Zusammensetzung nach Anspruch 1 oder 2, dadurch gekennzeichnet, daß das Medikament ist: Imipramin, Chlomimipramin, Zimelidin, Nortriptylin, Protriptylin, Amitriptylin, Fluxpenthixol, Butriptylin, Maprotilin, Nomifensin, Prothiaden, Fluphenazin, Desimipramin, Iprindol, Viloxazin, Dothiepin, Doxepin, Trimipramin, Mianserin, Norzimelidin, Phenelzin, Isocarboxizid, Tranylcypromin, Lithiumcarbonat, Chlorpromazin, Flupenthixol, Haloperidol, Thiordiazin, Thiothixen, Pimozid, Promazin oder Trifluoperazin.

4. Zusammensetzung nach einem der Ansprüche 1 bis 3, dadurch gekennzeichnet, daß sie in der Form einer Tablette oder Kapsel ist.

## Revendications

1. Une composition pharmaceutique comprenant de l'acide folique avec un médicament anti-dépresseur ou "anti-manique" autre que le tryptophane, dans une proportion d'acide folique de 100 à 300 µg par dose quotidienne du médicament.

2. Composition selon la revendication 1 contenant l'acide folique dans la proportion d'environ 200 µg par dose quotidienne du médicament.

3. Composition selon la revendication 1 ou 2, dans laquelle le médicament est l'imipramine, la chlomimipramine, la zimélidine, la nortriptyline, la protriptyline, l'amitriptyline, le fluxpenthixol, la butriphtyline, la maprotiline, la nomifensine, le prothiaden, la fluphénazine, la désimipramine, l'iprindole, la viloxazine, le dothiépine, la doxépine, la trimipramine, la miansérine, la norzimélidine, la phénelzine, l'isocarboxizide, l'iproniazide, la tranylcypromine, le carbonate de lithium, la chlorpromazine, le flupenthixol, l'halopéridol, la thioridazine, le thiothixène, le pimozide, la promazine ou la trifluopérazine.

4. Composition selon l'une quelconques des revendications 1 à 3 sous la forme d'un comprimé ou d'une capsule.